# EUROPEAN PATENT APPLICATION

(11) **EP 4 317 406 A1**
(43) Date of publication of application: **07.02.2024**
(21) Application number: 21934644.2
(22) Date of filing: 07.12.2021
(51) Int. Cl.: C12M 1/36, C12M 1/12, C12M 1/00

(54) **AUTOMATIC SLIDING TABLE MECHANISM OF CULTURE VESSEL CARRIER AND SHAKER INCUBATOR**

(30) Priority: 29.03.2021 CN 202120622984 U; 29.03.2021 CN 202120623168 U
(71) Applicant: Innovel Intelligent Technology Co., Ltd., Suzhou, Jiangsu 215000 (CN); Wuxi Biologics Ireland Limited, Dundalk, Co Louth A91 X56F (IE)
(72) Inventor: CHEN, Hao, Suzhou, Jiangsu 215000 (CN); TAN, Kee Wee, Shanghai 200131 (CN); DING, Jie, Shanghai 200131 (CN)
(74) Representative: Modiano, Gabriella Diana
(86) International application number: PCT/CN2021/136103
(87) International publication number: WO 2022/206008

(57) **Abstract**

The present application belongs to the technical field of biopharmaceutical production devices, and discloses a culture vessel carrier automatic slide mechanism and a shaker incubator, by means of which a shake flask/shake tube/well plate carrier assembly can automatically slide in and out relative to an incubator main body. The culture vessel carrier automatic slide mechanism comprises a slide protective housing and is provided with a culture vessel carrier bearing member. A first slide rail and a second slide rail are arranged below the culture vessel carrier bearing member. The second slide rail is arranged below the culture vessel carrier bearing member and located above the first slide rail, and the first slide rail is arranged inside the incubator main body on a fixed base that does not slide. An intermediate slide rail fixing plate secured to an inner wall of the slide protective housing is connected between the first slide rail and the second slide rail, and a sliding channel is provided on each side of the intermediate slide rail fixing plate. During sliding out and in, the sliding channels on the intermediate slide rail fixing plate can synchronously slide with the second slide rail, and the second slide rail can slide relative to the intermediate slide rail fixing plate, so as to achieve two-stage sliding of a bearing tray of the shake flask/shake tube/well plate carrier assembly arranged on the slide protective housing.

## Description

### Technical Field

The present invention relates to the technical field of biopharmaceutical production devices, and in particular to a culture vessel carrier automatic slide mechanism for use in an automated shaker incubator to allow a culture vessel carrier for carrying a culture vessel to automatically slide out to facilitate operations such as automatic sampling and adding of culture media, and a shaker incubator having the culture vessel carrier automatic slide mechanism.

### Background Art

Biopharmaceuticals are in a period of rapid development. macromolecular drugs such as antibodies are developing at a high speed. In the process of research and development and production of macromolecular drugs such as antibodies, a large number of shake flasks, shake tubes, well plates and other consumables for carrier and target product cultures are used, and shaker incubators are necessary production apparatus.

In a traditional shaker incubator, operations such as observation, testing, sampling, and adding of culture media are mainly carried out manually by the staff. The shaker incubator is difficult to interface with a newly developed automated transfer system, a liquid operation center, and intelligent scheduling and management software, may not be adapted to a rapid and automated research and development and production process of mass biological drugs, and is also unable to realize intelligent data acquisition, storage and transmission in the production process.

Therefore, in order to improve the automation of the traditional shaker incubator, there is an urgent need for a culture vessel carrier automatic slide mechanism to alleviate the above problems.

### Disclosure of the Invention

### Technical problems to be solved by the invention

An objective of the present invention is to provide a culture vessel carrier automatic slide mechanism and a shaker incubator having the culture vessel carrier automatic slide mechanism, which solve the problems mentioned in background art above.

### Technical solutions employed to solve the technical problems

In order to achieve the above objective, one aspect of the present invention provides a culture vessel carrier automatic slide mechanism, comprising a slide protective housing, a culture vessel carrier bearing member being mounted in the middle of the top of an inner wall of the slide protective housing in a left-right direction, and the culture vessel carrier bearing member being configured for placement of a bearing tray of a shake flask/shake tube/well plate carrier assembly thereon, characterized in that a two-stage sliding part including a first slide rail and a second slide rail is arranged on each side in the left-right direction below the culture vessel carrier bearing member, the second slide rail is arranged below the culture vessel carrier bearing member and located above the first slide rail, the first slide rail is arranged inside an incubator body of a shaker incubator on a fixed base that does not slide, an intermediate slide rail fixing plate secured to the inner wall of the slide protective housing is connected between the first slide rail and the second slide rail, and a sliding channel is provided on each side of the intermediate slide rail fixing plate that is close to the first slide rail and the second slide rail, a slide mechanism drive motor for driving the culture vessel carrier automatic slide mechanism is provided below the culture vessel carrier bearing member, and a drive gear is coupled to an output shaft side of the slide mechanism drive motor, two synchronous pulleys, i.e., a front synchronous pulley and a rear synchronous pulley, are arranged in a front-rear direction of the intermediate slide rail fixing plate on a side on which the drive gear is arranged in the left-right direction below the culture vessel carrier bearing member, a synchronous belt is hung between the front synchronous pulley and the rear synchronous belt, a front synchronous belt press block capable of forming an integral structure with the first slide rail and pressing the synchronous belt from below is arranged on a side of the synchronous belt that is close to the front synchronous pulley below the intermediate slide rail fixing plate, and a rear synchronous belt press block capable of forming an integral structure with the second slide rail and pressing the synchronous belt from above is arranged on a side of the synchronous belt that is close to the rear synchronous pulley above the intermediate slide rail fixing plate, a rack capable of meshing with the drive gear is mounted on a side on which the drive gear is arranged of the inner wall of the slide protective housing in the left-right direction, the slide mechanism drive motor in the culture vessel carrier automatic slide mechanism is powered on automatically when an automatic door of a shaker incubator is opened or closed, so as to drive the drive gear to rotate, the rack moves relative to the drive gear of the slide mechanism drive motor to cause the second slide rail and the intermediate slide rail fixing plate to synchronously move in a slide-out direction or a slide-in direction, such that a first stage of sliding of the bearing tray of the shake flask/shake tube/well plate carrier assembly arranged on the slide protective housing is achieved, thereafter or at the same time, the front synchronous pulley and the rear synchronous pulley arranged on the intermediate slide rail fixing plate move in the slide-out direction or slide-in direction to drive the synchronous belt to rotate, as the front synchronous belt press block and the rear synchronous belt press block press the synchronous belt from below and above, the second slide rail slides out towards the front relative to the sliding channels of the intermediate slide rail fixing plate due to the effect of the static friction of the synchronous belt on the rear synchronous belt press block, such that a second stage of sliding of the bearing tray of the shake flask/shake tube/well plate carrier assembly arranged on the slide protective housing is achieved.

Another aspect of the present invention provides a shaker incubator, comprising an incubator main body, comprising an incubator main body, wherein an automatic seal door assembly is arranged on a front side of the incubator main body, the automatic seal door assembly has an automatic door capable of being opened and closed automatically based on system control, and a shake flask/shake tube/well plate carrier assembly having a bearing tray and a shake flask/a shake tube/well plate carried on the bearing tray, and an intelligent integrated control system unit for controlling actions of various automatic components in the shaker incubator are arranged inside the incubator main body, wherein the culture vessel carrier automatic slide mechanism as described above is further arranged inside the incubator main body, the culture vessel carrier automatic slide mechanism enables, by means of the intelligent integrated control system unit, the shake flask/shake tube/well plate carrier assembly to automatically slide out relative to the incubator main body after the automatic door of the automatic seal door assembly is opened, and enables the shake flask/shake tube/well plate carrier assembly to automatically slide into the incubator main body before the automatic door of the automatic seal door assembly is closed.

### Effects of the invention

According to the construction as described above, the culture vessel carrier automatic slide mechanism of the present invention has the following beneficial effects compared to the prior art.
1. With the culture vessel carrier automatic slide mechanism arranged inside the incubator main body, the shake flask/shake tube/well plate carrier assembly can automatically slide in and out relative to the incubator main body to seamlessly interface with an automatic transfer system.
2. In the culture vessel carrier automatic slide mechanism, as the rack moves relative to the drive gear of the slide mechanism drive motor, the second slide rail and the intermediate slide rail fixing plate synchronously move in the sliding-out direction or the sliding-in direction, such that the first stage of sliding-out of the bearing tray of the shake flask/shake tube/well plate carrier assembly is achieved. Thereafter or at the same time, the second slide rail slides out towards the front relative to the sliding channels of the intermediate slide rail fixing plate due to the effect of the static friction of the synchronous belt on the rear synchronous belt press block, such that the second stage of sliding-out of the culture vessel carrier automatic slide mechanism is achieved. In this way, a secondary extension of stroke is ultimately achieved to allow the culture vessel carrier automatic slide mechanism to slide out over a long distance in a limited space, thereby enabling the bearing tray of the shaker flask/shake tube/well plate carrier assembly to slide completely out of the incubator main body of the shaker incubator, which facilitates automated operations of an external truss robot.
3. When the slide protective housing slides out using the culture vessel carrier automatic slide mechanism, the two supporting portions do not slide out relative to the incubator main body with the slide protective housing, while the two supporting wheels slide out relative to the incubator main body with the slide protective housing. Thereby, after the slide protective housing slides out, a front end of the slide protective housing is supported on the automatic door by the two supporting wheels, and a rear end is supported by the two supporting portions that do not follow the sliding out, such that hydrostatic balancing of the automatic slide mechanism in a sliding-out state can be achieved without cantilever phenomenon.

Furthermore, according to the construction as described above, the shaker incubator of the present invention has the following beneficial effects compared to the prior art,
1. With the automation design of the shaker incubator, and particularly the above-mentioned culture vessel carrier automatic slide mechanism, the automatic seal door assembly and the shaker mechanism drive unit, it is possible to realize fully automatic operations such as automatic opening of the door of the shaker incubator, automatic sliding-out of the bearing tray, automatic sampling and releasing, automatic sliding-in of the bearing tray, automatic closing of the door, and automatic operating and stopping of a shaker, thereby reducing manpower costs and reducing human operation errors and risks.
2. With the overall design of the automated shaker incubator, integration with an automatic production system is enabled, which allows for large-scale deployment and a significant increase in production capacity and throughput.
3. With the overall design of the automated shaker incubator, integration with an information system is enabled, so as to allow for automatic uploading and storage of various types of data, which facilitates product tracking and also ensures the integrity and traceability of data.

### Brief Description of the Drawings

FIG. 1 is a front view schematically representing an automated shaker incubator of the present invention.
FIG. 2 is a partial sectional perspective view schematically representing an internal structure of an incubator main body of the automated shaker incubator of the present invention.
FIG. 3 is a schematic front view representing the internal structure of the incubator main body of the automated shaker incubator of the present invention viewed from the front.
FIG. 4 is a schematic top view representing the internal structure of the incubator main body of the automated shaker incubator of the present invention viewed from the top.
FIG. 5 is a schematic perspective view illustrating representing a specific internal structure of an automatic slide mechanism of the present invention.
FIG. 6 is a side view representing the interior of the automatic slide mechanism of the present invention shown in FIG. 5 viewed from a side.
FIG. 7 is a schematic diagram representing a structure of a slide mechanism drive motor of the automatic slide mechanism of the present invention.
FIG. 8 is a flow block diagram of automated operations implemented by the automated shaker incubator of the present invention.

### Detailed Description of Embodiments

Technical solutions in the embodiments of the present invention will be described below with reference to the accompanying drawings in the embodiments of the present invention. Apparently, the described embodiments are merely some rather than all of the embodiments of the present invention. On the basis of the embodiments of the present invention, all other embodiments derived by those of ordinary skill in the art without any creative efforts fall within the scope of protection of the present invention.

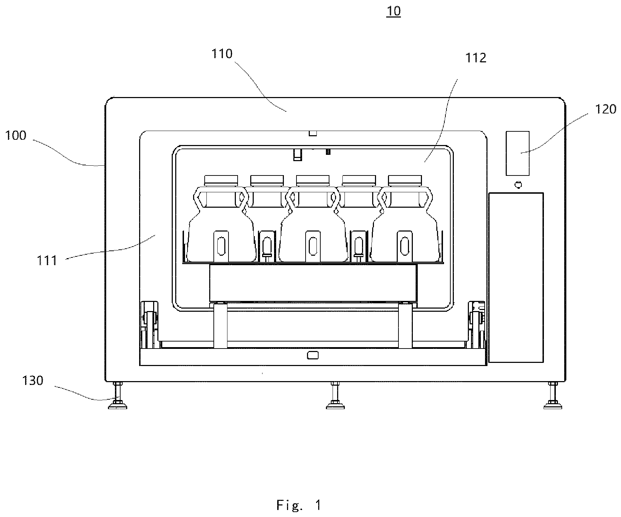

First, an automated shaker incubator 10 of the present invention is described in detail with reference to FIGS. 1-4.

The automated shaker incubator 10 of the present invention shown in FIG. 1 comprises an incubator main body 100. An automatic seal door assembly 110 is arranged on a front side of the incubator main body 100. The automatic seal door assembly 110 comprises: an automatic door 111 capable of being opened and closed automatically based on system control; and, for example, a door opening and closing drive motor, a driving gear and a driven gear that are configured for realizing automatic opening and closing of the automatic door 111.

In this embodiment, an observation window 112 is arranged on a front side of the automatic door 111. The automatic door 111 is connected to the incubator main body 100 of the shaker incubator 10, for example, by means of engagement. Furthermore, in this embodiment, although not shown in the figures, the driving gear is arranged at a drive end of the door opening and closing drive motor, and the driven gear is connected to the automatic door 111. The driving gear is connected to the driven gear by means of engagement. However, the present invention is not limited thereto. The automatic opening and closing of the automatic door 111 may be realized, for example, by means of transmission between the driving gear, the driven gear and a belt, or by means of extension and retraction of a connecting rod.

In addition, a visual operating part 120 is arranged on the front side of the incubator main body 100 and located on a side (e.g., an upper right side in FIG. 1) of the automatic seal door assembly 110, the visual operating part 120 is, for example, a touch display that can realize both mode operations and visual current-state confirmation. However, the present invention is not limited thereto. The visual operating part may also be a combination of a button for realizing only mode operations and a display for realizing only current-state visual confirmation.

Furthermore, a plurality of (e.g., six) supporting legs 130 are arranged at the bottom of the shaker incubator 10. FIG. 1 shows three supporting legs 130 on the front side. However, the present invention is not limited thereto. It is possible to provide no supporting legs, or to provide a different number of supporting legs from six, as long as it is possible to ensure the stability of the shaker incubator 10 during operations.

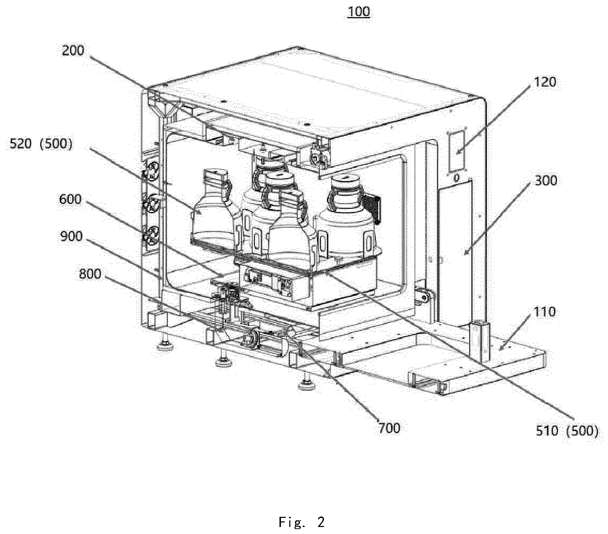

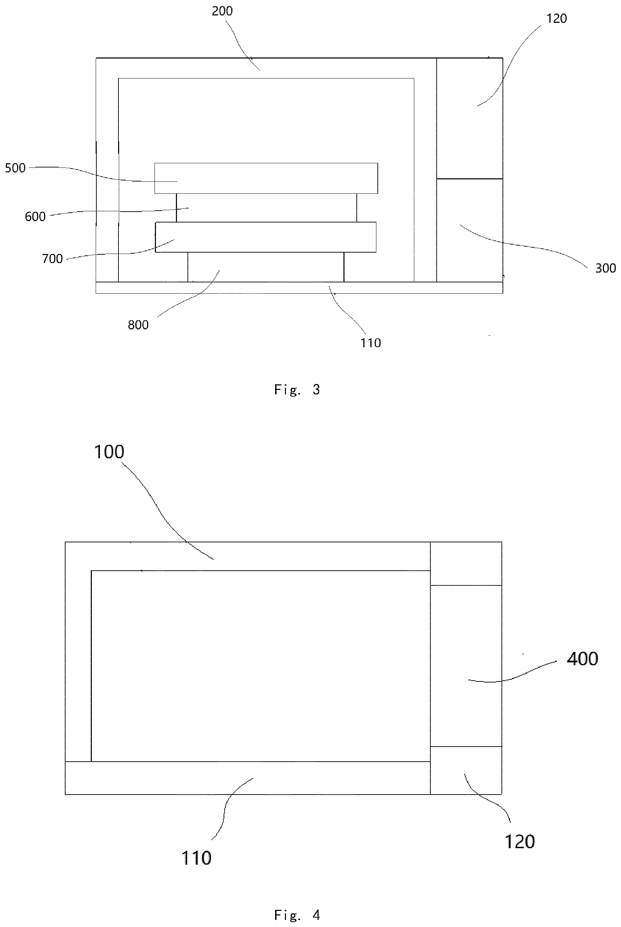

FIG. 2 is a partial sectional perspective view schematically representing an internal structure of the incubator main body 100 of the automated shaker incubator 10 of the present invention. FIG. 3 is a schematic front view representing the internal structure of the automated shaker incubator 10 of the present invention viewed from the front. FIG. 4 is a schematic top view representing the internal structure of the automated shaker incubator 10 of the present invention viewed from the top.

As shown in FIGS. 2, 3, and 4, the incubator main body 100 of the shaker incubator 10 includes therein: a device illumination/purification/disinfection unit 200, the apparatus illumination/purification/disinfection unit 200 having an ultraviolet lamp and a fluorescent lamp for illuminating, purifying and disinfecting the interior of the incubator main body 100; an intelligent integrated control system unit 300, the intelligent integrated control system unit 300 having a PLC controller, a data storage chip, a data transmission device, and a remote control device and being configured to control actions of various automatic components inside the incubator main body 100; an environment control unit 400, the environment control unit 400 having a temperature sensor, a humidity sensor and a light sensor for controlling environmental parameters such as temperature, humidity and light of the interior of the incubator main body 100; a shake flask/shake tube/well plate carrier assembly 500 (also referred to as "a culture vessel carrier"), the shake flask/shake tube/well plate carrier assembly 500 having a bearing tray 510 and a shake flask/shake tube/well plate 520 (also referred to as "a culture vessel") carried on the bearing tray 510; a culture vessel carrier automatic slide mechanism 600, wherein the culture vessel carrier automatic slide mechanism 600 enables the shake flask/shake tube/well plate carrier assembly 500 to automatically slide out relative to the incubator main body 100 after the automatic door 111 of the automatic seal door assembly 110 is opened, and enables the shake flask/shake tube/well plate carrier assembly 500 to automatically slide into the incubator main body 100 before the automatic door 111 of the automatic seal door assembly 110 is closed; a shaker mechanism 700, wherein the shaker mechanism 700 may be a usual shaker mechanism having a rotating disc and a fastener; and a shaker mechanism drive unit 800, the shaker mechanism drive unit 800 having a shaker mechanism drive motor and a connecting base connected to the incubator main body 100.

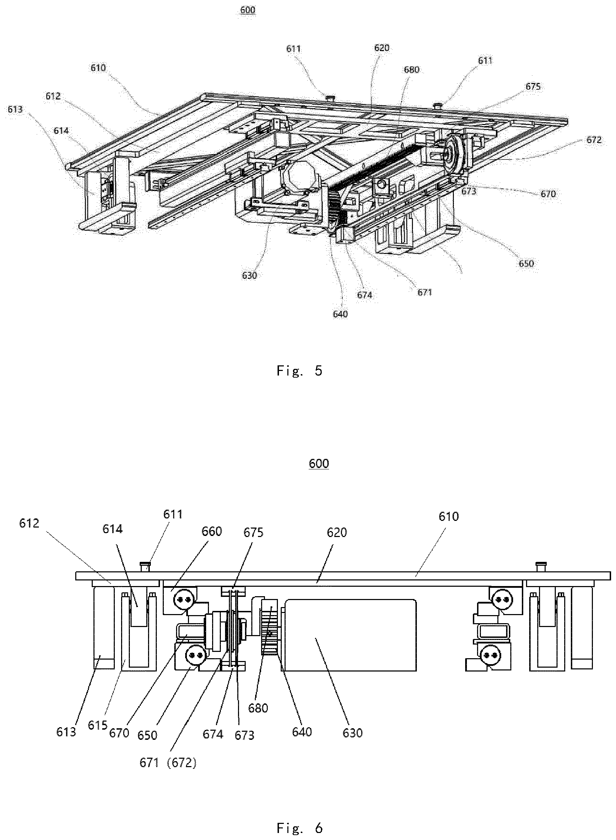

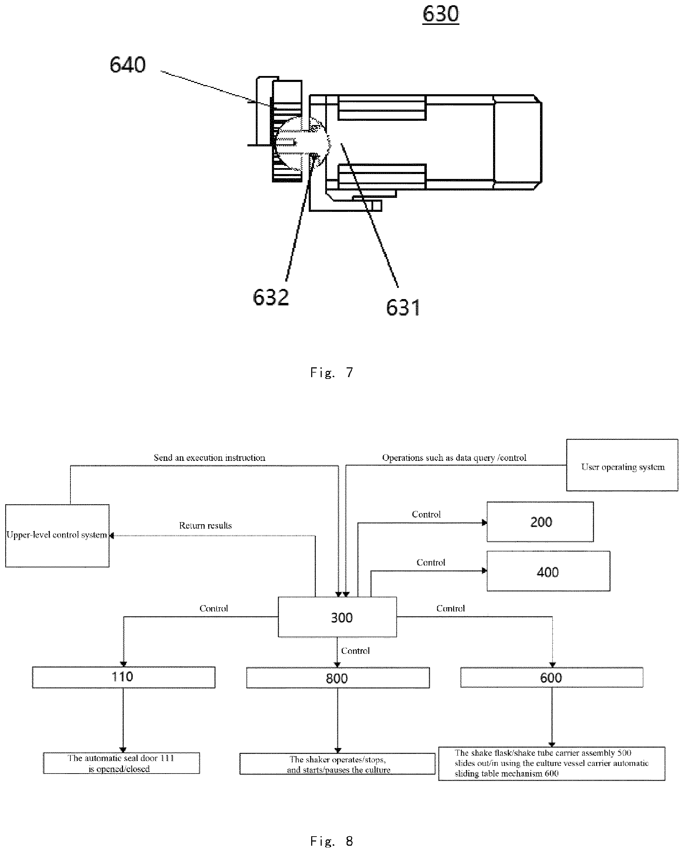

Hereinafter, the structure of the culture vessel carrier automatic slide mechanism 600 of the present invention inside the incubator main body 100 is described in detail with reference to FIGS. 5, 6, and 7. FIG. 5 is a schematic perspective view representing a specific internal structure of the culture vessel carrier automatic slide mechanism 600 of the present invention. FIG. 6 is a side view representing the interior of the culture vessel carrier automatic slide mechanism 600 of the present invention shown in FIG. 5 viewed from a side. FIG. 7 is a schematic diagram representing a structure of a slide mechanism drive motor of the automatic slide mechanism of the present invention.

As shown in FIG. 5, the culture vessel carrier automatic slide mechanism 600 inside the incubator main body 100 of the fully automated shaker incubator 10 according to an embodiment of the present invention comprises a slide protective housing 610, with a plurality of (two exemplified in FIG. 6) culture vessel carrier positioning pins 611 distributed in a left-right symmetrical manner, for example, on a side (e.g., the back side in FIG. 5) of a base surface of the slide protective housing 610 in a front-rear direction. In addition, although not shown in the figure, a plurality of culture vessel carrier positioning holes are correspondingly provided in the shake flask/shake tube/well plate carrier assembly 500. By aligning the culture vessel carrier positioning pins 611 of the culture vessel carrier automatic slide mechanism 600 to the culture vessel carrier positioning holes of the shake flask/shake tube/well plate carrier assembly 500, the shake flask/shake tube/well plate carrier assembly 500 can be accurately positioned and arranged on the slide protective housing 610 of the culture vessel carrier automatic slide mechanism 600. Thereby, as the culture vessel carrier automatic slide mechanism 600 slides out or in relative to the incubator main body 100 when the automatic door 111 is opened or closed, the shake flask/shake tube/well plate carrier assembly 500 positioned and arranged on the culture vessel carrier automatic slide mechanism 600 can also slide out or in relative to the incubator main body 100 together.

As shown in FIGS. 5 and 6, a culture vessel carrier bearing member 620 is mounted in the middle of the top of an inner wall of the slide protective housing 610 in the left-right direction. The culture vessel carrier bearing member 620 is configured for placement of the bearing tray 510 of the shake flask/shake tube/well plate carrier assembly 500 thereon. Supported portions 612 are arranged on two sides of the inner wall of the slide protective housing 610 in the left-right direction at positions corresponding to two outermost culture vessel carrier positioning pins 611 in the left-right direction, the supported portions 612 are supported, in the front-rear direction, by two supporting portions 613 that are arranged on the outermost sides (the leftmost side and the rightmost side), in the left-right direction, of the culture vessel carrier automatic slide mechanism 600, and the two supporting portions 613 have the same structure and are fixed to the bottom of the incubator main body 100 of the shaker incubator 10 in the front-rear direction. Supporting wheels 614 are fixedly arranged respectively at parts of the supported portions 612 inside of the supporting portions 613 (i.e., the right side of the leftmost supporting portion 613 and the left side of the rightmost supporting portion 613) in the left-right direction, and guide grooves 615 for positioning and sliding of the supporting wheels 614 therein are provided below the supporting wheels 614.

As shown in FIGS. 5 and 6, a slide mechanism drive motor 630 for driving the culture vessel carrier automatic slide mechanism 600 is arranged below the culture vessel carrier bearing member 620. As shown in FIG. 7, an output shaft of the speed reducer 631 runs through a sealed housing of the slide mechanism drive motor 630, a V-shaped seal ring 632 is mounted at a joint between the speed reducer 631 and the sealed housing of the slide mechanism drive motor 630, and the output shaft of the speed reducer 631 is coupled to a drive gear 640 by means of a coupling. The slide mechanism drive motor 630 is a bi-directional rotary motor capable of rotating in forward and reverse directions and is powered on and rotates in different directions in response to the opening and closing of the automatic seal door assembly 110. In addition, the V-shaped seal ring 632 is made of a silicone material.

A two-stage sliding part including a first slide rail 650 and a second slide rail 660 is arranged inside of each of the supporting wheels 614 on two sides (e.g., the right side of the supporting wheel 614 located on the left side and on the left side of the supporting wheel 614 located on the right side in FIG. 6) in the left-right direction below the culture vessel carrier bearing member 620. The second slide rail 660 is arranged below the culture vessel carrier bearing member 620 and located above the first slide rail 650, the first slide rail 650 is arranged inside the incubator main body 100 of the shaker incubator 10 on a fixed base (e.g., a device frame portion 900 inside the incubator main body 100 of the shaker incubator 10) that does not slide. Furthermore, an intermediate slide rail fixing plate 670 secured to the inner wall of the slide protective housing 610 is connected between the first slide rail 650 and the second slide rail 660, and a sliding channel is provided on a side of the intermediate slide rail fixing plate 670 that is close to each of the first slide rail 650 and the second slide rail 660 (not illustrated).

Since the second slide rail 660 are arranged below the culture vessel carrier bearing member 620 and the intermediate slide rail fixing plate 670 is secured to the inner wall of the slide protective housing 610, the sliding channels on the intermediate slide rail fixing plate 670 are capable of sliding synchronously with the second slide rail 660 when the entirety of the slide protective housing 610 slides out and in. Moreover, the second slide rail 660 can slide relative to the intermediate slide rail fixing plates 670. Furthermore, since the first slide rail 650 is arranged at the bottom of the incubator main body 100 of the shaker incubator 10 that does not slide, the sliding channels on the intermediate slide rail fixing plates 670 slide only relative to the first slide rail 650 when the entirety of the slide protective housing 610 slides out and in.

Furthermore, as shown in FIGS. 5 and 6, two synchronous pulleys, i.e., a front synchronous pulley 671 and a rear synchronous pulley 672, are arranged in a front-rear direction of the intermediate slide rail fixing plate 670 on a side on which the drive gear 640 is arranged (e.g., the left side of the slide mechanism drive motor 630 in FIG. 6) in the left-right direction below the culture vessel carrier bearing member 620, and a synchronous belt 673 is hung between the front synchronous pulley 671 and the rear synchronous belt 672. The intermediate slide rail fixing plate 670 on the other side (e.g., the right side of the drive motor 630 of the slide mechanism in FIG. 6) in the left-right direction below the culture vessel carrier bearing member 620 can be provided with synchronous pulleys and a synchronous belt or with no synchronous pulleys or synchronous belt. For the purpose of simplifying the structure, it is preferred that the synchronous pulleys and the synchronous belt are not provided, and that only the sliding channels of the intermediate slide rail fixing plate 670 on the right side are utilized to slide relative to the first slide rail 650 and the second slide rail 660 on the right side.

As shown in FIG. 5, a front synchronous belt press block 674 capable of forming an integral structure with the first slide rail 650 and pressing the synchronous belt 673 from below is arranged on a side of the synchronous belt 673 that is close to the front synchronous pulley 671 below the intermediate slide rail fixing plate 670, and a rear synchronous belt press block 675 capable of forming an integral structure with the second slide rail 660 and pressing the synchronous belt 673 from above is arranged on a side of the synchronous belt 673 that is close to the rear synchronous pulley 672 above the intermediate slide rail fixing plate 670.

In addition, as shown in FIG. 5, a rack 680 capable of meshing with the drive gear 640 is mounted on a side on which the drive gear 640 is arranged (e.g., the left side of the slide mechanism drive motor 630 in FIG.6), of the inner wall of the slide protective housing 610 in the left-right direction. When the automatic seal door assembly 110 is opened, the slide mechanism drive motor 630 is powered on to rotate in one direction, causing the drive gear 640 coupled to the output shaft of the speed reducer 631 to rotate in one direction, and causing the rack 680 meshing with the drive gear 640 to move towards the front relative to the slide mechanism drive motor 630, and the culture vessel carrier automatic slide mechanism 600 (the slide protective housing 610) moves towards the front (the first stage of sliding-out ) accordingly.

In this case, as the slide protective housing 610 moves towards the front, the second slide rail 660 arranged below the culture vessel carrier bearing member 620 and the intermediate slide rail fixing plates 670 secured to the slide protective housing 610 synchronously move towards the front, such that the front synchronous pulley 671 and the rear synchronous pulley 672 arranged on the intermediate slide rail fixing plate 670 move towards the front, causing the synchronous belt 673 hung between the front synchronous pulley 671 and the rear synchronous belt 672 and close to a side (the upper side) of the second slide rail 660 to rotate towards the front and causing the synchronous belt 673 close to a side (the lower side) of the first slide rail 650 to rotate towards the rear. Furthermore, since the synchronous belt 673 is pressed by the front synchronous belt press block 674 from below and pressed by the rear synchronous belt press block 675 from above, the rear synchronous belt press block 675 moves towards the front with the synchronous belt 673 on the upper side under the effect of the static friction of the synchronous belt 673 on the rear synchronous belt press block 675, such that the second slide rail 660 which forms an integral structure with the rear synchronous belt press block 675 further slides out towards the front relative to the sliding channels of the intermediate slide rail fixing plate 670 (the second stage of sliding-out).

In other words, in the culture vessel carrier automatic slide mechanism 600 of the present invention, as the rack 680 moves towards the front relative to the drive gear 640 of the slide mechanism drive motor 630, the second slide rail 660 and the intermediate slide rail fixing plates 670 synchronously move towards the front, such that the first-stage of sliding-out of the culture vessel carrier automatic slide mechanism 600 is achieved. Thereafter or at the same time, the front synchronous pulley 671 and the rear synchronous pulley 672 arranged on the intermediate slide rail fixing plate 670 move towards the front to drive the synchronous belt 673 to rotate, since the front synchronous belt press block 674 and the rear synchronous belt press block 675 press the synchronous belt 673 from below and above, the second slide rail 660 slides out towards the front relative to the sliding channels of the intermediate slide rail fixing plate 670 due to the effect of the static friction of the synchronous belt 673 on the rear synchronous belt press block 675, such that the second stage of sliding-out of the culture vessel carrier automatic slide mechanism 600 is achieved. In this way, a secondary extension of stroke is ultimately achieved to allow the culture vessel carrier automatic slide mechanism 600 to slide out over a long distance in a limited space, thereby enabling the bearing tray 510 of the shaker flask/shake tube/well plate carrier assembly 500 to slide completely out of the incubator main body 100 of the shaker incubator 10.

Furthermore, in the automated shaker incubator 10 of the present invention, with the culture vessel carrier automatic slide mechanism 600, the shake flask/shake tube/well plate carrier assembly 500 (and the bearing tray 510) is allowed to automatically slide in and out to interface with an automatic transfer system seamlessly.

Furthermore, when the slide protective housing 610 slides out using the culture vessel carrier automatic slide mechanism 600, two supporting portions 613, two guide grooves 615, two first slide rails 650, the slide mechanism drive motor 630, and the drive gear 640 shown in FIG. 6 do not slide out relative to the incubator main body 100 with the slide protective housing 610, while two supporting wheels 614, two second slide rails 650, two intermediate slide rail fixing plates 670, etc. slide out relative to the incubator main body 100 with the slide protective housing 610. Particularly, after the slide protective housing 610 slides out, a front end of the slide protective housing 610 is supported on the automatic door 111 by the two supporting wheels 614, and a rear end is supported by the two supporting portions 613 that do not follow the sliding out, such that hydrostatic balancing of the automatic slide mechanism in a sliding-out state can be achieved without cantilever phenomenon.

Finally, an automated workflow of the automated shaker incubator 10 of the present invention is described with reference to FIG. 8. FIG. 8 is a flow block diagram of automated operations implemented by the automated shaker incubator 10 of the present invention.

After receiving an operation instruction from a user operating system about data query/control and the like, the intelligent integrated control system unit 300 inside the incubator main body 100 of the automated shaker incubator 10 of the present invention sends the instruction to an upper-level control system, and the upper-level control system sends an execution instruction to the intelligent integrated control system unit 300. The intelligent integrated control system unit 300 is specifically capable of controlling the device illumination/purification/disinfection unit 200, the environment control unit 400, the automatic seal door assembly 110, the shaker mechanism drive unit 800, and the culture vessel carrier automatic slide mechanism 600, and feeding results back to the upper-level control system.

Furthermore, in the automated shaker incubator 10 of the present invention, the automatic components include the automatic seal door assembly 110, the shaker mechanism drive unit 800, and the culture vessel carrier automatic slide mechanism 600. However, the present invention is not limited thereto, and automatic components with other functions may be integrated therein.

The automatic seal door assembly 110 can be automatically opened and closed under the control of the intelligent integrated control system unit 300. The shaker mechanism drive unit 800 can make the shaker operate and stop, and start and pause the culture under the control of the intelligent integrated control system unit 300. Furthermore, the culture vessel carrier automatic slide mechanism 600 enables the automatic sliding in and out of the shake flask/shake tube/well plate carrier assembly 500 (and the bearing tray 510) under the control of the intelligent integrated control system unit 300.

A workflow of the present invention is as follows. In use of the automated shaker incubator 10, firstly, an operator replaces a corresponding bearing tray according to a production system planning, and sets the device operation and environment control parameters according to the production system planning by using the touch visual operating part 120. Thereafter, the shaker incubator 10 starts to carry out pre-culture related operations such as self-checking, sterilization and residue discharging in the interior of the incubator main body 100, and then the automatic door 111 of the shaker incubator 10 is opened and the culture vessel carrier automatic slide mechanism 600 slides out. An external truss robot is used to place shake flasks containing culture solution into the bearing tray 510 of the shake flask/shake tube/well plate carrier assembly 500 in sequence. The culture vessel carrier automatic slide mechanism 600 slides into the interior of the incubator main body 100, the automatic door 111 of the shaker incubator 10 is closed, and the shaker mechanism 700 is started to perform a shaking culture process. During the culture, the device automatically records all corresponding data, and automatically uploads the data according to the system settings or saves the data locally, and then the incubator system, according to the upper-level system planning, regularly carries out operations such as sampling for observation, adding of culture media, and replacement of shake flasks. Finally, after a culture cycle is finished, respective parts of the incubator are cleaned and maintained. The whole operation process is simple and convenient. Compared with an existing shaker incubator, the shaker incubator of the present invention can improve the overall convenience, intelligence and practicality by virtue of the design.

Although the embodiments of the present invention have been shown and described, it can be understood by those of ordinary skill in the art that various changes, modifications, substitutions and variations can be made to these embodiments without departing from the principles and spirit of the present invention, and the scope of the present invention is defined by the appended claims and their equivalents.

### (List of reference signs)

- 10: Shaker incubator;
- 100: Incubator main body;
- 110: Automatic seal door assembly;
- 111: Automatic door;
- 112: Observation window;
- 120: Visual operating part;
- 130: Supporting leg;
- 200: Device illumination/purification/disinfection unit;
- 300: Intelligent integrated control system unit;
- 400: Environment control unit;
- 500: Shake flask/shake tube/well plate carrier assembly;
- 510: Bearing tray;
- 520: Shake flask/shake tube/well plate;
- 600: Culture vessel carrier automatic slide mechanism;
- 610: Slide protective housing;
- 611: Culture vessel carrier positioning pin;
- 612: Supported portion;
- 613: Supporting portion;
- 614: Supporting wheel;
- 615: Guide groove;
- 620: Culture vessel carrier bearing member;
- 630: slide mechanism drive motor;
- 631: Speed reducer;
- 632: V-shaped seal ring;
- 640: Drive gear;
- 650: First slide rail;
- 660: Second slide rail;
- 670: Intermediate slide rail fixing plate;
- 671: Front synchronous pulley;
- 672: Rear synchronous pulley;
- 673: Synchronous belt;
- 674: Front synchronous belt press block;
- 675: Rear synchronous belt press block;
- 680: Rack;
- 700: Shaker mechanism;
- 800: Shaker mechanism drive unit.

## Claims

1. A culture vessel carrier automatic slide mechanism (600), comprising a slide protective housing (610), a culture vessel carrier bearing member (620) being mounted in the middle of the top of an inner wall of the slide protective housing (610) in a left-right direction, and the culture vessel carrier bearing member (620) being configured for placement of a bearing tray (510) of a shake flask/shake tube/well plate carrier assembly (500) thereon,
**characterized in that**
a two-stage sliding part including a first slide rail (650) and a second slide rail (660) is arranged on each side in the left-right direction below the culture vessel carrier bearing member (620), the second slide rail (660) is arranged below the culture vessel carrier bearing member (620) and located above the first slide rail (650), the first slide rail (650) is arranged inside an incubator main body (100) of a shaker incubator (10) on a fixed base that does not slide, an intermediate slide rail fixing plate (670) secured to the inner wall of the slide protective housing (610) is connected between the first slide rail (650) and the second slide rail (660), and a sliding channel is provided on a side of the intermediate slide rail fixing plate (670) that is close to each of the first slide rail (650) and the second slide rail (660),
a slide mechanism drive motor (630) for driving the culture vessel carrier automatic slide mechanism (600) is arranged below the culture vessel carrier bearing member (620), and a drive gear (640) is coupled to an output shaft side of the slide mechanism drive motor (630),
two synchronous pulleys, i.e., a front synchronous pulley (671) and a rear synchronous pulley (672), are arranged in a front-rear direction of the intermediate slide rail fixing plate (670) on a side on which the drive gear (640) is arranged in the left-right direction below the culture vessel carrier bearing member (620), a synchronous belt (673) is hung between the front synchronous pulley (671) and the rear synchronous belt (672), a front synchronous belt press block (674) capable of forming an integral structure with the first slide rail (650) and pressing the synchronous belt (673) from below is arranged on a side of the synchronous belt (673) that is close to the front synchronous pulley (671) below the intermediate slide rail fixing plate (670), and a rear synchronous belt press block (675) capable of forming an integral structure with the second slide rail (660) and pressing the synchronous belt (673) from above is arranged on a side of the synchronous belt (673) that is close to the rear synchronous pulley (672) above the intermediate slide rail fixing plate (670),
a rack (680) capable of meshing with the drive gear (640) is mounted on a side on which the drive gear (640) is arranged of the inner wall of the slide protective housing (610) in the left-right direction, and
the slide mechanism drive motor (630) in the culture vessel carrier automatic slide mechanism (600) is powered on automatically when an automatic door (111) of a shaker incubator (10) is opened or closed, so as to drive the drive gear (640) to rotate, the rack (680) moves relative to the drive gear (640) of the slide mechanism drive motor (630) to cause the second slide rail (660) and the intermediate slide rail fixing plate (670) to synchronously move in a slide-out direction or a slide-in direction, such that a first stage of sliding of the bearing tray (510) of the shake flask/shake tube/well plate carrier assembly (500) arranged on the slide protective housing (610) is achieved, thereafter or at the same time, the front synchronous pulley (671) and the rear synchronous pulley (672) arranged on the intermediate slide rail fixing plate (670) move in the slide-out direction or slide-in direction to drive the synchronous belt (673) to rotate, as the front synchronous belt press block (674) and the rear synchronous belt press block (675) press the synchronous belt (673) from below and above, the second slide rail (660) slides out towards the front relative to the sliding channels of the intermediate slide rail fixing plate (670) due to the effect of the static friction of the synchronous belt (673) on the rear synchronous belt press block (675), such that a second stage of sliding of the bearing tray (510) of the shake flask/shake tube/well plate carrier assembly (500) arranged on the slide protective housing (610) is achieved.

2. The culture vessel carrier automatic slide mechanism (600) according to claim 1, **characterized in that**
a speed reducer (631) is arranged inside a sealed housing of the slide mechanism drive motor (630), and
an output shaft of the speed reducer (631) runs through the sealed housing of the slide mechanism drive motor (630) and is coupled to the drive gear (640) by means of a coupling.

3. The culture vessel carrier automatic slide mechanism (600) according to claim 2, **characterized in that**
a V-shaped seal ring (632) is mounted at a joint between the speed reducer (631) and the sealed housing of the slide mechanism drive motor (630).

4. The culture vessel carrier automatic slide mechanism (600) according to claim 1, **characterized in that**
a plurality of culture vessel carrier positioning pins (611) are provided on a side of a base surface of the slide protective housing (610) in a front-rear direction, and the culture vessel carrier positioning pins (611) are aligned to a plurality of culture vessel carrier positioning holes correspondingly provided in the shake flask/shake tube/well plate carrier assembly (500), and the shake flask/shake tube/well plate carrier assembly (500) and the slide protective housing (610) are allowed to slide out or slide in relative to the incubator main body (100) of the shaker incubator (10) when the automatic door (111) of the shaker incubator (10) is opened or closed.

5. The culture vessel carrier automatic slide mechanism (600) according to claim 4, **characterized in that**
supported portions (612) are arranged on two sides of the inner wall of the slide protective housing (610) in the left-right direction at positions corresponding to two outermost culture vessel carrier positioning pins (611) of the plurality of the culture vessel carrier positioning pins (611) in the left-right direction,
the supported portions (612) are supported, in the front-rear direction, by two supporting portions (613) that are arranged on the outermost side, in the left-right direction, of the culture vessel carrier automatic slide mechanism (600), and the two supporting portions (613) have the same structure and are fixed to the fixed base in the front-rear direction.

6. The culture vessel carrier automatic slide mechanism (600) according to claim 5, **characterized in that**
supporting wheels (614) are fixedly arranged respectively at parts of the supported portions (612) inside of the supporting portions (613) in the left-right direction, and
guide grooves (615) for positioning and sliding of the supporting wheels (614) therein are provided below the supporting wheels (614).

7. The culture vessel carrier automatic slide mechanism (600) according to claim 6, **characterized in that**
the supporting portions (613), the guide grooves (615), the first slide rail (650), the slide mechanism drive motor (630), and the drive gear (640) are not capable of sliding out relative to the incubator main body (100) with the slide protective housing (610),
the supporting wheels (614), the second slide rail (650), and the intermediate slide rail fixing plate (670) are capable of sliding out relative to the incubator main body (100) with the slide protective housing (610), and
when the automatic door (111) of the shaker incubator (10) is opened and the slide protective housing (610) is caused to slide out by means of the culture vessel carrier automatic slide mechanism (600), a front end of the slide protective housing (610) that slides out is supported on the automatic door (111) by the supporting wheels (614), and a rear end of the slide protective housing (610) that slides out is supported by the supporting portions (613) that do not follow the sliding out.

8. The culture vessel carrier automatic slide mechanism (600) according to claim 1, **characterized in that**
the slide mechanism drive motor (630) is a bi-directional rotary motor capable of rotating in forward and reverse directions and is powered on and rotates in different directions in response to the opening and closing of the automatic seal door assembly (110).

9. A shaker incubator (10), comprising an incubator main body (100), wherein an automatic seal door assembly (110) is arranged on a front side of the incubator main body (100), the automatic seal door assembly (110) has an automatic door (111) capable of being opened and closed automatically based on system control, and
a shake flask/shake tube/well plate carrier assembly (500) having a bearing tray (510) and a shake flask/a shake tube/well plate (520) carried on the bearing tray (510), and an intelligent integrated control system unit (300) for controlling actions of various automatic components in the shaker incubator (10) are arranged inside the incubator main body (100),
**characterized in that**
the culture vessel carrier automatic slide mechanism (600) of any one of claims 1 to 8 is further arranged inside the incubator main body (100), the culture vessel carrier automatic slide mechanism (600) enables, by means of the intelligent integrated control system unit (300), the shake flask/shake tube/well plate carrier assembly (500) to automatically slide out relative to the incubator main body (100) after the automatic door (111) of the automatic seal door assembly (110) is opened, and enables the shake flask/shake tube/well plate carrier assembly (500) to automatically slide into the incubator main body (100) before the automatic door (111) of the automatic seal door assembly (110) is closed.

10. The shaker incubator (10) according to claim 9, **characterized in that**
a visual operating part (120) is arranged on the front side of the incubator main body (100) and located on a side of the automatic seal door assembly (110), the visual operating part (120) is a member capable of simultaneously realizing both mode operations and visual current-state confirmation, or a combination of a member for realizing only mode operations and a member for realizing only current-state visual confirmation.

11. The shaker incubator (10) according to claim 9, **characterized in that**
the automatic seal door assembly (110) comprises a door opening and closing drive motor, a driving gear and a driven gear that are configured for realizing automatic opening and closing of the automatic door (111),
the driving gear is arranged at a drive end of the door opening and closing drive motor, and
the driven gear connected to the driving gear is connected to the automatic door (111).

12. The shaker incubator (10) according to claim 9, **characterized in that** further provided inside the interior of the incubator main body (100) are:
a device illumination/purification/disinfection unit (200), the apparatus illumination/purification/disinfection unit (200) having an ultraviolet lamp and a fluorescent lamp for illuminating, purifying and disinfecting the interior of the incubator main body (100);
an environment control unit (400), the environment control unit (400) having a temperature sensor, a humidity sensor and a light sensor for controlling environmental parameters of the interior of the incubator main body (100);
a shaker mechanism (700), the shaker mechanism (700) having a rotating disc and a fastener; and
a shaker mechanism drive unit (800), the shaker mechanism drive unit (800) having a shaker mechanism drive motor and a connecting base connected to the incubator main body (100).

13. The shaker incubator (10) according to claim 12, **characterized in that** in the interior of the incubator main body (100),
the automatic seal door assembly (110), the shaker mechanism drive unit (800) and the culture vessel carrier automatic slide mechanism (600) are provided as the automated components, and
the intelligent integrated control system unit (300) has a PLC controller, a data storage chip, a data transmission device and a remote control device.
